# EUROPEAN PATENT APPLICATION

(11) **EP 2 557 152 A1**
(43) Date of publication of application: **13.02.2013**
(21) Application number: 10849266.1
(22) Date of filing: 07.04.2010
(51) Int. Cl.: C12N 5/07, C12N 5/071, C12N 15/11

(54) **KEY GENES, MICRORNAS, OTHER NON-CODING RNAS AND COMBINATIONS THEREOF FOR IDENTIFYING AND REGULATING PLURIPOTENCY OF CELLS**

(71) Applicant: Institute Of Zoology, Chinese Academy Of Sciences, Chaoyang District, Beijing 100101 (CN); Institute Of Genetics And Developmental Biology, Chinese Academy Of Sciences, Beijing 100101 (CN)
(72) Inventor: ZHOU, Qi, Beijing 100101 (CN); WANG, Xiujie, Beijing 100101 (CN); WANG, Liu, Beijing 100101 (CN); LIU, Lei, Beijing 100101 (CN); ZHAO, Xiaoyang, Beijing 100101 (CN); YANG, Wei, Beijing 100101 (CN); LUO, Guanzheng, Beijing 100101 (CN); LV, Zhuo, Beijing 100101 (CN); ZHENG, Qinyuan, Beijing 100101 (CN); WU, Huajun, Beijing 100101 (CN); LI, Wei, Beijing 100101 (CN)
(74) Representative: Raukema, Age
(86) International application number: PCT/CN2010/071622
(87) International publication number: WO 2011/124029

(57) **Abstract**

The invention provides key genes, microRNAs, other non-coding RNAs and a combination thereof for identifying and regulating pluripotency of cells, characterized in that they are highly expressed in full pluripotent stem cells but are significantly suppressed or silenced in partially pluripotent stem cells. The genes, microRNAs and other non-coding RNAs are those of a chromosome imprinted Dlk1-Dio3 region located on the long arm of mouse chromosome 12, and are homologous genes, microRNAs and other non-coding RNAs having 70-100% homology with them in genomic syntenic regions of other mammals. The invention also provides uses of the genes, microRNAs, other non-coding RNAs and combination thereof in the invention in identifying the pluripotent status of stem cells and regulating pluripotency of cells; in typing stem cells; regulating pluripotency of cells, pluripotent states and levels of cells; treating diseases; and developing drug targets for tumor treatment and antitumor drugs.

## Description

### Technical Field of the Invention

The invention relates to key genes, microRNAs, or other non-coding RNAs or a combination thereof for identifying and regulating the pluripotent status of cells. The invention also relates to uses of the genes and non-coding microRNAs of the invention in identifying the pluripotent status of stem cells and in typing stem cells; regulating pluripotency of cells, pluripotent states and levels of cells; treating diseases; and developing drug targets for tumor treatment or antitumor drugs.

### Background of the Invention

Pluripotent cells are a type of cells having an ability of multiple differentiation potential to form various tissues and cells of human and animal individuals after cell division and cell differentiation. At present, Embryonic Stem Cell (ESC) is the most well known pluripotent cell. The ES cell is a special cell line obtained by culturing in *vitro* cells, which are isolated from the inner cell mass of blastocysts in an early stage of embryo development of mammals, under certain conditions. This type of cells has an ability of keeping undifferentiated state and infinite proliferation. Nowadays, the ES cells have been successfully induced to differentiate into nearly all types of cells, including nerve cells, hepatic cells, endothelial cells, cardiac muscle cells, pancreatic beta cells and hematopoietic cells. The development of stem cells and regenerative medicine plays an important promoting role in the field of development biology, tissue-organ transplantation, gene therapy, cell therapy and drug development, and will be expected to resolve the medical problems confronted by human beings, such as cardiac-cerebral vascular disease, diabetes and neurological disease; thus, the new breakthrough and development of bioscience and biotechnology caused thereby would bring revolutionary changes to the population health and the medical field and become the high-tech industry with the great potential in 21^{st} century.

The conventional ES cell encounters two bottlenecks not easily to be conquered in the application of regenerative medicine; first, the method of acquiring the ES cells needs to adopt an early embryo, which involves the ethical problem; second, when the ES cells acquired are used for a clinical patient, immunological rejection problems would occur. In order to overcome the problem of immunological rejection, the first problem people meet is how to reprogram somatic cells of a patient into pluripotent cells through various methods and then to differentiate into cells of needed types for cell therapy and organ transplantation. The appearance of a cloned sheep named Dolly in 1997 proved that ovocyte has the ability of reprogramming a differentiated mammalian adult cell into the state of embryonic full pluripotency and this cell can generate pluripotent stem cells and develop into a new individual. However, the lack of oocytes and the ethical problems involved in the use of embryos limit the development of this technology in the field of regenerative medicine. In 2006, Yamanaka first proved that somatic cells can be reprogrammed into pluripotent stem cells, that is, induced pluripotent stem cells (iPS cells) (Takahashi et al., 2007; Takahashi and Yamanaka, 2006; Yu et al., 2007), by means of the forced expression of four exogenous transcription factors. This discovery makes the research of somatic cell reprogramming down to the level of genes, and meanwhile broadened the direction of the reprogramming research. From the technical perspective, the iPS cells are derived from somatic cells but not an embryo, which avoids the ethical controversy confronted by human ES cells. The research of cell reprogramming has developed many important fields including somatic cell nuclear transplantation, iPS technology and other means, by using cell fusion, small molecule compounds induction and many research methods.

Over the years, the identification of the pluripotent status of ES cells depends on animal chimeric experiments; however, human chimeric experiments can not be implemented due to legal restrictions and ethical problems; therefore, the identification of the pluripotent status of human ES cells lacks a powerful standard. People are always trying to seek for proper markers to distinguish the developmental potentiality of stem cells; however, no success is achieved so far.

Totally reprogrammed iPS cells have true pluripotency, can differentiate into every type of somatic cells and can give birth to mouse with normal reproductive capacity through the method of tetraploid embryo complementation (Zhao et al., 2009). However, the screening of totally reprogrammed iPS cells through the method of tetraploid embryo complementation has a high technical requirement and the success rate is very low, which seriously delays the development and application of the iPS technology. Due to the restriction of ethics, the pluripotency of human iPS cells can not be validated through the method of tetraploid embryo complementation; thus, iPS cells meet a significant barrier when finally being applied to clinic; therefore, a new iPS cell classification standard is needed to screen the iPS cells with true pluripotency.

Aiming at the above problems, the invention first adopted the conventional pluripotency validation method of stem cells to divide different iPS cells into two types, from one type (hereinafter called 4N iPS cells) animals can be obtained through the method of tetraploid embryo complementation, and from the other type (hereinafter called 2N iPS cells) animals can be obtained only through the method of diploid chimera. Then the invention compared the expression difference of genes and non-coding RNAs between these two types of iPS cells through a gene chip and microRNA sequencing, and discovered a group of genes, microRNA clusters and other non-coding RNAs that are highly expressed in the 4N iPS cell but are not expressed or lowly expressed in the 2N iPS cell. This group of genes, microRNA clusters and other non-coding RNAs are located in an imprinted region (or imprinted domain) of chromosome 12, including five coding genes, three non-coding genes, a C/D box snoRNA gene cluster, a microRNA cluster containing a plurality of very conservative microRNAs, and some other non-coding RNAs. And the invention proved that this region not only reflects the pluripotent state of cells, but also determines the developmental potentiality of cells. This result has been validated in many cell lines from different sources. The chip/microarray designed for the non-coding microRNA of this region can serve as a standard of characterizing the developmental potentiality of pluripotent cells. Compared with the conventional method of tetraploid complementation, the technique of this invention has a lower technical requirement, a shorter operation period and does not involve an ethic factor, and has a broad application prospect in the field of iPS cells.

Since these genes and non-coding microRNAs are very conservative in mammals, the cluster of genes, microRNAs and other non-coding RNAs probably has the same important functions in human ES cells and other mammals. This achievement first proposes an effective marker for identifying the pluripotent status of cells, and may adjust the pluripotent state and level of cells by regulating this cluster of genes, revolutionarily change the stem cells and iPS technology and change the international research pattern of stem cells, obtain a new breakthrough in the research of pathogenesis of diseases such as tumour, discover potential drug targets for tumour treatment and develop new antitumor drugs. In addition, this invention has not screened out other gene clusters which can definitely distinguish the pluripotent state of cells, on other locations of the mouse genome; thus, the coding genes, microRNA clusters and other non-coding RNAs in this imprinted region probably are the unique key identifier for determining the pluripotent state of cells; therefore, it has enormous significance..

### Summary of the Invention

Since for the first time to prove the full pluripotency of iPS cells, the inventors first discovered and definitely verified the key genes, microRNA clusters and other non-coding RNAs for characterizing the pluripotent status of mouse cells. These genes, microRNA clusters and other non-coding RNAs are located on chromosome 12 and are a very conservative microRNA gathering region; cells in which the genes and non-coding RNAs of this region are highly expressed show full differentiation potential, while the expression of this region is significantly suppressed or silenced in cells with partial differentiation pluripotency; this region not only reflects the pluripotent state of cells, but also determines the developmental potentiality of cells. Since the coding microRNAs of this region only exist in the genomes of mammals and are very conservative, the cluster of microRNAs probably has the same important function in mammals including human ES cells. This achievement for the first time proposes an effective marker for identifying the pluripotent status of cells, the pluripotent state and level of cells may be adjusted by regulating this cluster of genes, and a new breakthrough may be obtained in the research of pathogenesis of diseases such as tumour, potential drug targets for tumour treatment may be discovered and new antitumor drugs may be developed.

The inventors find that the key genes, microRNAs, other non-coding RNAs and a combination thereof for identifying and regulating the pluripotent status of cells are highly expressed in full pluripotent stem cells but are significantly suppressed or silenced in partially pluripotent stem cells.

The expression level of the key genes, microRNAs, other non-coding RNAs and combination thereof in the invention is higher in the pluripotent stem cells which can produce germ-line chimeric mice than in the pluripotent stem cells which can not produce germ-line chimeric mice.

The genes, microRNAs, other non-coding RNAs and combination thereof in the invention are characterized in that the genes, microRNAs and other non-coding RNAs include all genes, microRNAs and other non-coding RNA of a chromosome imprinted Dlk1-Dio3 region (containing Dlk1 and Dio3 genes and the intergenic regions from this region to upstream gene Begain and to downstream gene Ppp2r5c) located on the long arm of mouse chromosome 12, and homologous genes, microRNAs and other non-coding RNA having 70% or more than 70% homology with them in polynucleotide sequences in genomic synteny regions of other mammals. The chromosome imprinted Dlk1-Dio3 region is of about 1380kb. The genes, microRNAs, other non-coding RNAs and combination thereof in the invention are characterized in that the microRNA in the invention includes all microRNAs listed in SEQ ID 1-72.

The invention contains homologous genes, microRNAs and other non-coding RNA of the above region in the genomic syntenic regions of other mammals.

The genomic region contained in the invention includes five genes of Dlk1, Rtl1, 1110006E14Rik, B830012L14Rik and Dio3, three non-coding genes of Meg3, Rian and Mirg, a gene cluster of C/D box snoRNA, a microRNA cluster and a plurality of other non-coding RNAs.

The invention also relates to uses of the genes, microRNAs, other non-coding RNAs and combination thereof in the invention in identifying the pluripotent status of stem cells or regulating the pluripotent status of cells; regulating the pluripotent status of cells, pluripotent states and levels of cells and typing stem cells; treating diseases; and developing drug targets for tumor treatment.

The microRNA cluster in the invention is highly conserved in mammals. In both humans and mice, the abnormal expression of imprinted genes of this region would cause the abnormal development of some embryos and placentas. This demonstrates that the functions of this cluster of microRNAs are conservative in mammals. Therefore, the inventors predict that this cluster of microRNAs has the same important functions in the human ES cells.

So far, the limited pluripotency and weak differentiation ability of human induced Pluripotent Stem (hiPS) cells is a serious problem (Hu et al.); besides, the pluripotency of human ES cells still can not be examined, and there lacks an effective means for distinguishing the ES cells and the iPS cells with different developmental potentialities. Since the expression of microRNA clusters of this region has an important influence on the development of embryo, the pluripotent status of human ES cells can be distinguished by detecting the expression of the microRNAs of this region, therefore to select the pluripotent cell with the high expression of microRNAs of this region, that is, with high pluripotent status and highest developmental potentiality level, for applications such as cell treatment. In addition, since the microRNA cluster of this region is highly conservative in mammals, the identification of pluripotent cells of other big animals also can be implemented by detecting the expression of these microRNAs, then, the validation work of pluripotent cells can be greatly simplified.

The inventors can identify the pluripotent status of ES cells and induced Pluripotent Stem Cells (iPSC) of mouse, rat, human and other big animals by detecting the expression of microRNAs of this region.

### Brief Description of the Drawings

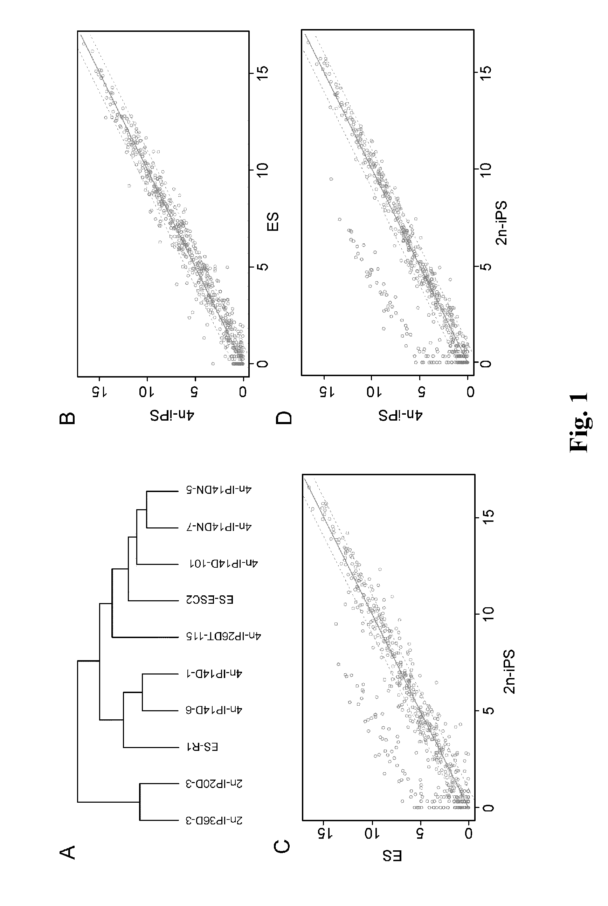

Fig. 1 shows that the expression of microRNA clusters in the invention has a difference in stem cell lines with full pluripotency and stem cell lines with partial pluripotency. A shows a cluster analysis of a microRNA sequencing result of ten pluripotent stem cells. B shows that the expression of the microRNAs has no obvious difference in ES cells and 4n-iPS cells. C shows that the expression of the microRNA cluster in the invention has an obvious difference in ES cells and 2n-iPS cells (marked by red circles). D shows that the expression of the microRNA cluster in the invention has an obvious difference in 4n-iPS cells and 2n-iPS cells. In each of the three cell lines of ES, 4n-iPS and 2n-iPS, the original clone number of the microRNA is normalized and an average value is obtained which then is converted into a base-2 logarithmic value for drawing a figure.

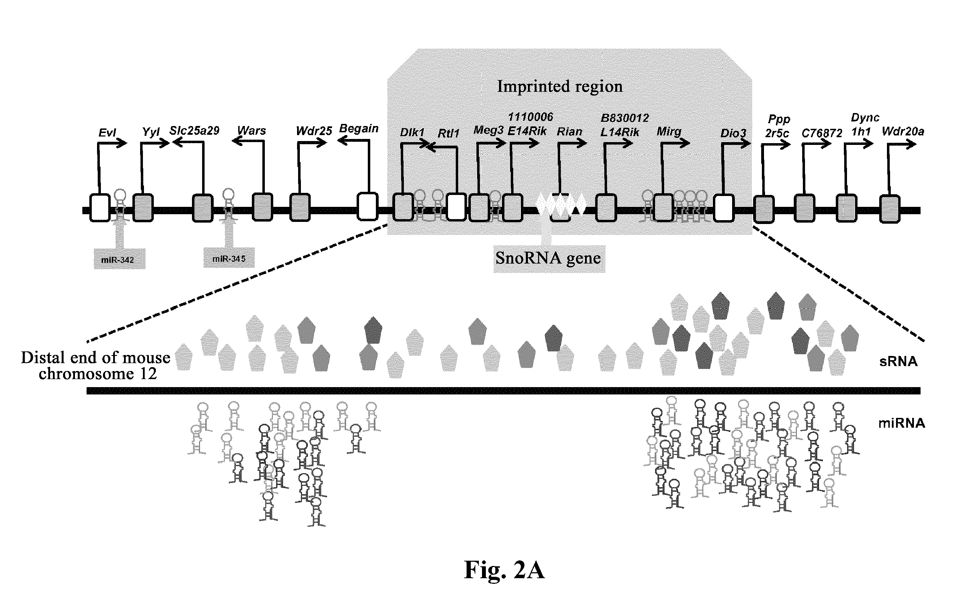

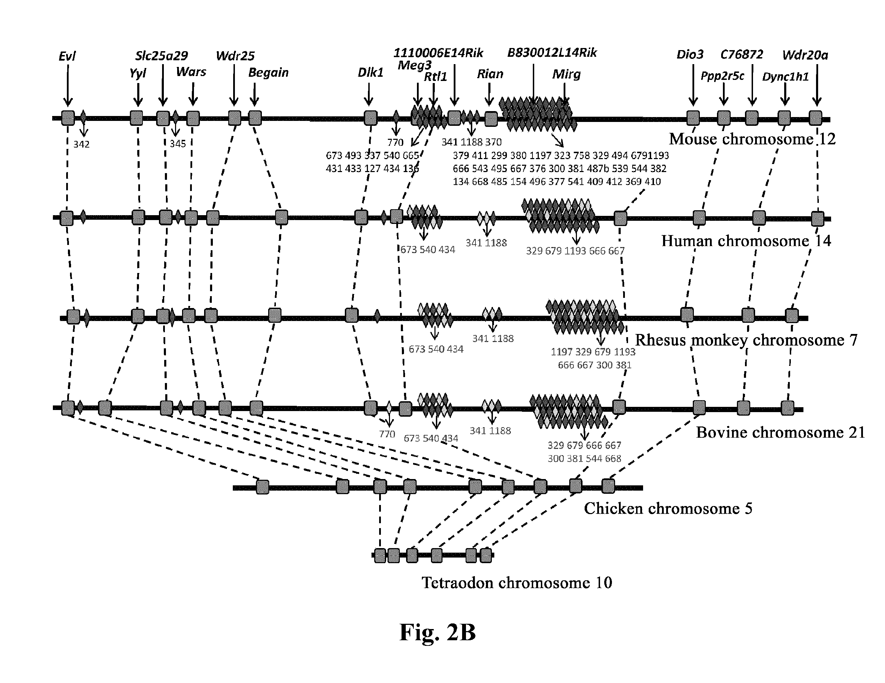

Fig. 2 shows expression and conservation conditions of the genome Dlk1-Dio3 region in the invention and the upstream and downstream adjacent genes thereof. A. The expression difference of fully pluripotent cells (ES and 2n-iPS) and partially pluripotent cells (4n-iPS) in the Dlk1-Dio3 region and adjacent region thereof. Compared with the partially pluripotent cells, in the fully pluripotent cells, the genes with high expression are marked by green rectangles (that is, grey rectangles in the imprinted region of a black-and white graph); the genes with moderate expression are marked by grey rectangles; and the genes not contained in the detection are marked by white rectangles. Hairpin patterns indicate microRNAs; pentagons indicate other shorter non-coding RNAs, wherein the shade of the lines and fill colours of the hairpin pattern and the pentagon are positively correlated to the expression level. B shows the conservative conditions of the Dlk1-Dio3 region and the upstream and downstream adjacent genes thereof. Small red diamonds indicate conservative microRNAs; small pink diamonds indicate unconservative microRNAs; green boxes indicate other genes. MicroRNA clusters only exist in mammals and are highly conserved in sequence.

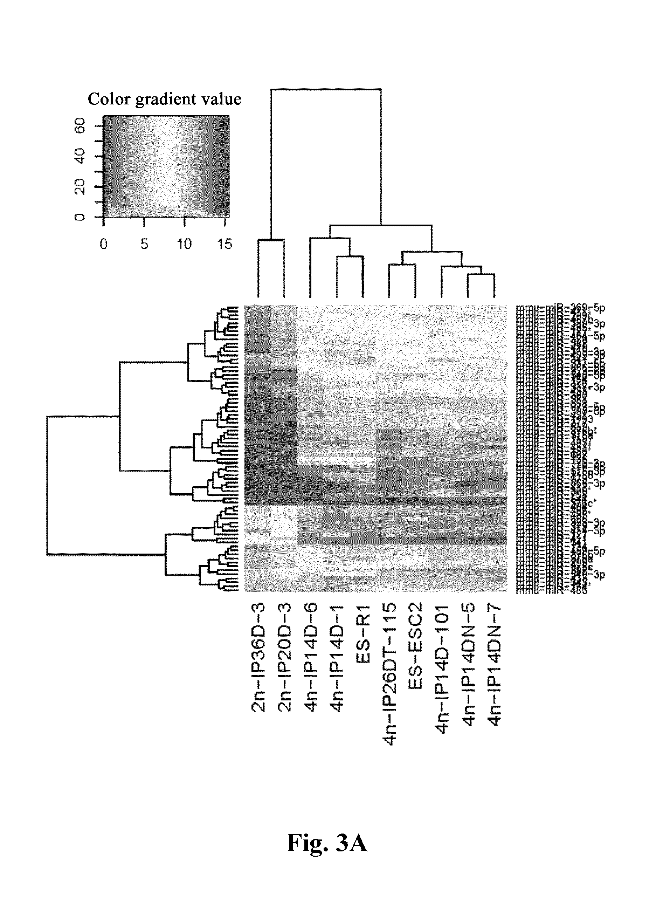

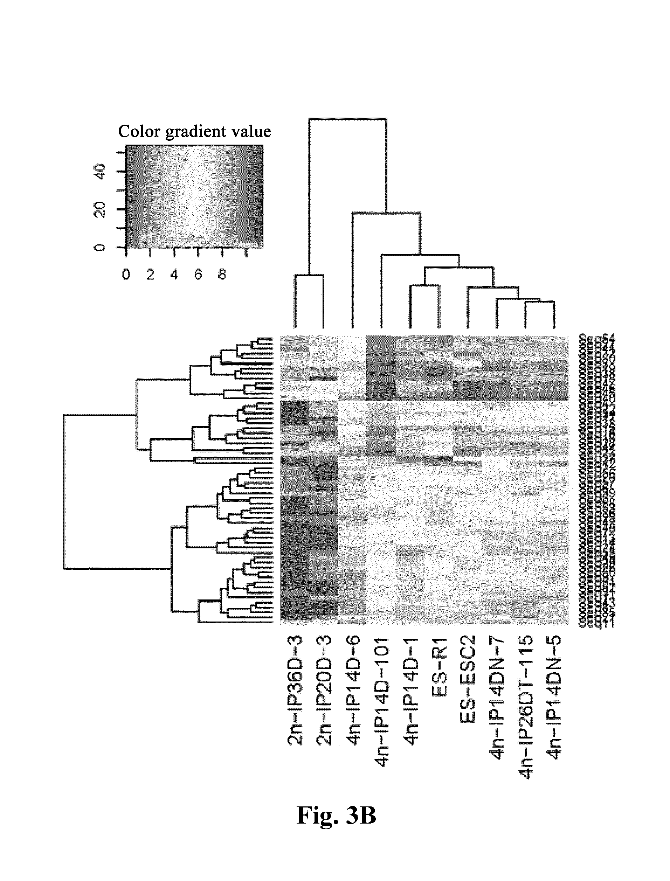

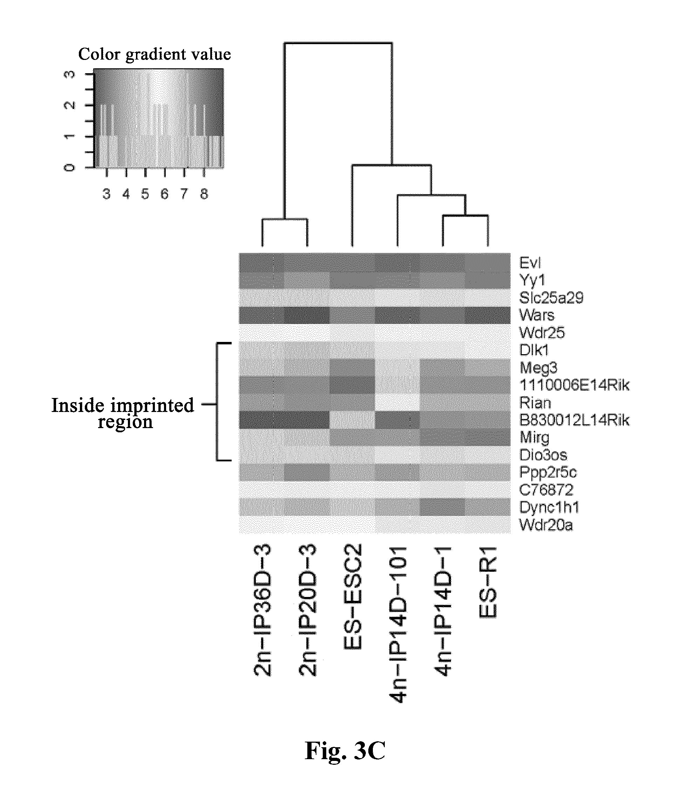

Fig. 3 shows a cluster analysis of expression levels of microRNAs (A), other non-coding small RNAs (B) and protein coding-genes (C) of the genomic Dlk1-Dio3 region. C also shows expression conditions of genes adjacent to the core Dlk1-Dio3 region. The expression value adopts an SOLEXA sequencing read number and a base-2 logarithmic value converted from an original signal value of an expression profile chip. The dendrogram is drawn by the hclust package in R.

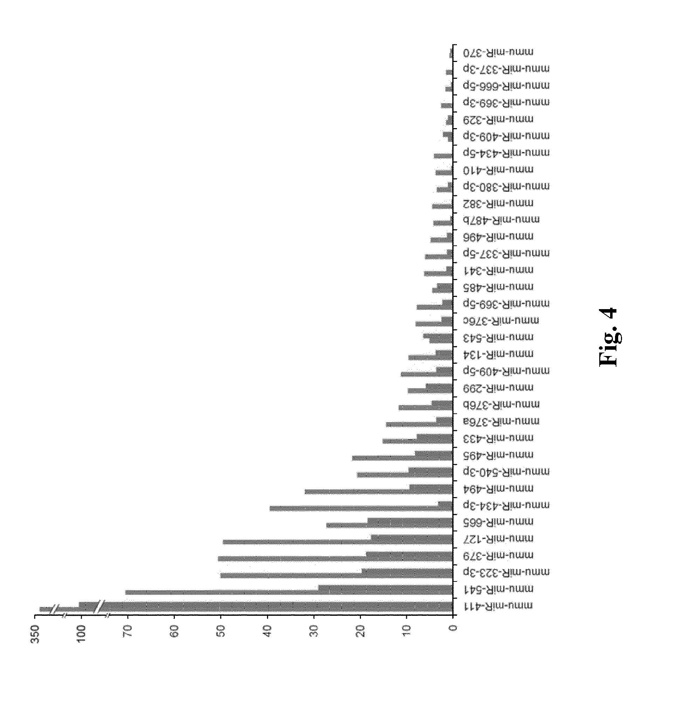

Fig. 4 shows that the expression level of microRNAs of the core Dlk1-Dio3 region has a difference between a 2n-iPS cell line with germ-line chimeric ability and a 2n-iPS cell line without germ-line chimeric ability. In the 2n-iPS cell line IP20D-3 with germ-line chimeric ability, the expression condition of microRNAs is marked by a blue column; in the 2n-iPS cell line IP36D-3 without germ-line chimeric ability, the expression condition of microRNAs is marked by a red column.

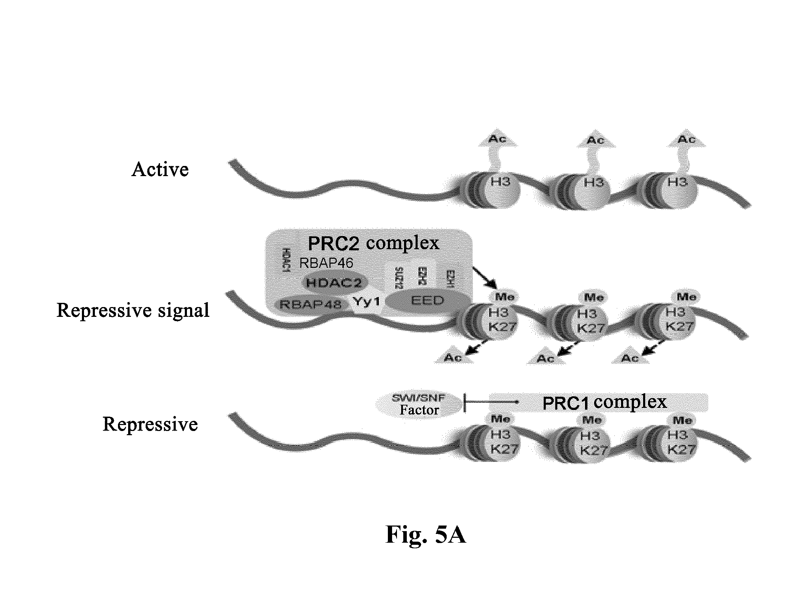

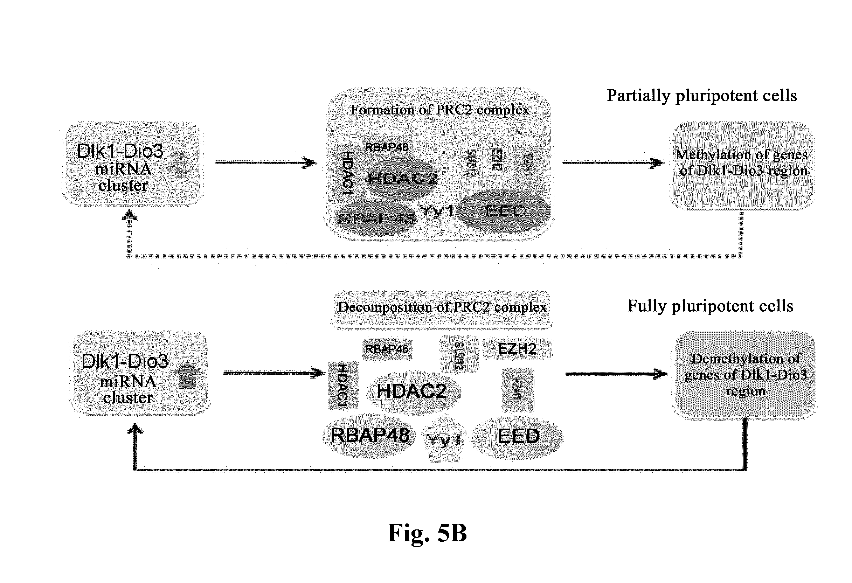

Fig. 5 shows a mechanism sketch model of microRNAs of the Dlk1-Dio3 region regulating a PRC2 complex. A shows that the PRC2 complex suppresses the expression of genes and microRNAs through tri-methylated-Histone H3K27 locus. B shows that, in fully pluripotent stem cells, the expression of microRNAs of the Dlk1-Dio3 region inhibits the formation of PRC2 complex by suppressing three proteins in the PRC2 complex, thereby facilitating the expression of microRNAs and other genes of the Dlk1-Dio3 region.

### Detailed Description of the Invention

The research of the inventors is based on rigorous animal experiments, identifies and classifies ES cells and pluripotent stem cells with different developmental potentialities, and traces the difference between these pluripotent stem cells through massive Solexa sequencing and gene chip data, and thus discovers that genes, microRNAs and other non-coding RNAs located in the Dlk1-Dio3 region are highly expressed in fully pluripotent cells with highest developmental potentiality but are significantly suppressed or silenced in partially pluripotent stem cells with lower developmental potentiality; this conclusion totally coincides with the result, which is obtained by first detecting the cluster of genes and non-coding microRNAs and then validating pluripotency. Therefore, in mice, the expression of the cluster of genes is an important marker for the pluripotent status of stem cells.

MicroRNA (miRNA) is a short-chain RNA has a length of an average of 22 nucleotides, which has a hairpin-shaped secondary structured precursor, can be complementary to the sequence of a corresponding target messenger RNA (mRNA) and regulate gene silencing at translational level post-transcription.

Materials and methods:

### 1. iPS cell line and pluripotent status identification

At present, the standards for judging the developmental potentiality of pluripotent stem cells of mice include: 1. expression of pluripotency markers, for example, pou5f1, Nanog, Rex1, etc.; 2. formation of Embryoid Body (EB) and teratoma; proving that it can differentiate into three germ layers; 3. inducing to differentiate into a plurality of cell types *in vitro,* for example, nerve cells, cardiac muscle cells, islet cells, etc.; 4. obtaining chimeric animals (2N); 5. capable of obtaining germ-line chimeric animals, that is, it can develop into a germ-line and into a sperm or egg (GLT); 6. healthy birth of tetraploid complementation animals (4N); the latter is the strictest standards (gold standards); however, due to ethical problems, only the former three standards are applicable to the judgement of human pluripotent stem cells. The inventors selected the fourth, fifth and sixth levels of stem cells of mice to conduct experiments.

This experiment first analysed the expression of pluripotent genes and cell karyotype according to a conventional method; then performed *in vitro* and *in vivo* examination experiments, including the formation of EB and teratoma, the formation of chimera and the tetraploid embryo complementation assays. The inventors injected the pluripotent stem cells into a CD-1 diploid mouse embryo to obtain chimeric mice; when these mice grew up, they were mated with CD-1 mice to produce infant mice; the production of germ-line chimeric mice was determined by their fur colors. The tetraploid embryo complementation is the strictest standard for detecting the pluripotent status of cells and the inventors obtained mice totally from the ES cell or iPS cell source by injecting pluripotent stem cells into tetraploid embryos.

The following cell lines were selected to extract total RNA and perform sequencing: 1. two ES cell lines and six iPS cell lines (4n) with the ability for germ-line chimerism and tetraploid complementation; 2. one iPS cell line (GLT) only with the germ-line chimeric ability; 3. one iPS cell line (2n) without germ-line chimeric ability but can produce chimera animals.

### 2. Extraction of total RNA of cells and detection of the completeness of RNA

The total RNA of cells were extracted using trizol (invitrogen); the extracted total RNA was detected for completeness through Agilent 2100, wherein the qualified RNA should satisfy RIN value being greater than or equal to 8.0, and the amount of 28s:18s being greater than or equal to 1.

### 3. High-throughput sequencing of small RNAs

### 3.1 Isolation of non-coding small RNAs

10ug of qualified total RNA was taken and isolated on denatured PAGE gel of 15% polyacrylamide (7M carbamide); small RNAs with lengths between 18-30nt were recovered by gel extraction.

### 3.2 Connection to 5' end joint

The recovered non-coding small RNAs were connected to the 5' end joint; the connection product was isolated on the denatured PAGE gel of 15% polyacrylamide (7M carbamide); 40-60nt of connection product was recovered by gel extraction.

### 3.3 Connection to 3' end joint

The recovered connection product was connected to the 3' end joint gain; the connection product was isolated on the denatured PAGE gel of 15% polyacrylamide (7M carbamide); 70-90nt of connection product was recovered by gel extraction.

### 3.4 RT-PCR

The recovered connection product was subjected to inverse transcription through specific primers to obtain cDNA, wherein the cDNA was subjected to PCR amplification for 15 amplification cycles and the product obtained by amplification was isolated on non-denatured PAGE gel of 10% polyacrylamide; 90bp of specific band was recovered by gel extraction.

### 3.5 High-throughput sequencing

Sequencing of the recovered PCR product was performed through a high-throughput sequencing instrument of Illumina Company.

### 4. Data analysis

Original data returned from the Illumina Company was obtained; the data were primarily processed using biological information methods; reads with sequencing quality not meeting standards were removed to obtain high-quality non-redundant sequences and expression values thereof. The sequences were located to the genome through sequence alignment; between pluripotent stem cells with different pluripotent status (based on whether a tetraploid complementation animal can be obtained), specific non-coding microRNA clusters were compared and screened by segments according to the location on the genome, to obtain features capable of indicating whether ES cells or iPS cells meet the standards of highest tetraploid-complementation pluripotency.

### 5. Gene expression profiling microarray and differentially expressed gene analysis

The expression profiling microarray hybridization results of all cell lines was processed using the bio-chip data and genome data analysis software package Bioconductor in R language environment. The original hybridization signal value of the chip was normalized by the RMA method and then converted into base-2 logarithmic values. In conjunction with FDR calibration, genes having expression differences between two cell lines were screened using Student's t-test (p<0.05). A thermograph of expressions of studied genes and non-coding microRNAs in the imprinted region was drawn using the software package "heatmap.2 package" in R.

### Results:

This research finds that the cluster of genes, microRNAs and other non-coding RNAs specifically expressed in mammals have significant effects on the pluripotency of mouse ES cells. All genes, microRNAs and other non-coding RNAs in the imprinted Dlk1-Dio3 region of mouse chromosome 12 (containing Dlk1 and Dio3 genes and intergenic regions from this region to the upstream gene Begain and to the downstream gene Ppp2r5c) are highly expressed in fully pluripotent stem cells with the ability to produce animals through tetraploid complementation, but are suppressed or silenced in partially pluripotent stem cells without the ability to produce animals through tetraploid complementation.

### iPS cell line and pluripotency identification

The iPS cells and ES cells used in the experiments were examined for the expression of pluripotent genes, karyotype analysis, EB and teratoma formation, diploid chimeric formation ability and tetraploid embryo complementation ability ; and the detection results are shown in Table 1.

**Table 1: Characterization of pluripotent stem cells**

| Pluripotent Cell | Donor Cell | Genetic Background | Expression of Pluripotency Markers (Oct4, Nanog, SSEA-1) Immunostaining | Karyotype | Embryoid Body | Teratoma | Chimera (Germ-Line Chimeric) | Tetraploid Complementation |
|---|---|---|---|---|---|---|---|---|
| IP36D-3 | Fetal Fibroblast | B6xD2 FI | √ | Normal | √ | √ | √(x) | x |
| IP20D-3 | Fetal Fibroblast | B6xD2 FI | √ | Normal | √ | √ | √(√) | x |
| ESC2 | Fertilized Egg | B6xD2 FI | √ | Normal | √ | √ | √(√) | √ |
| R1 | Fertilized Egg | 129X1/SvJ x129S1 F1 | √ | Normal | √ | √ | √(√) | √ |
| IP14D-1 | Fetal Fibroblast | B6xD2 FI | √ | Normal | √ | √ | √(√) | √ |
| IP14D-6 | Fetal Fibroblast | B6xD2 FI | √ | Normal | √ | √ | √(√) | √ |
| IP14D-101 | Fetal Fibroblast | B6x129S2 F1 | √ | Normal | √ | √ | √(√) | √ |
| IP26DT-1 5 | Tail Tip Fibroblast | B6x129S2 F1 | √ | Normal | √ | √ | √(√) | √ |
| IP14DN-5 | Neural Stem Cell | B6xD2 F1 | √ | Normal | √ | √ | √(√) | √ |
| IP14DN-7 | Neural Stem Cell | B6xD2 F1 | √ | Normal | √ | √ | √(√) | √ |

| | | | | | | | | |
|---|---|---|---|---|---|---|---|---|
| Note: √ means Yes; x means No. | | | | | | | | |

### The deep sequencing of non-coding microRNAs shows that a cluster of microRNAs is differently expressed in stem cells with different developmental potentialities

In order to find a key indicator for judging whether the pluripotent stem cells have the tetraploid complementation ability at molecular level, 18nt to 30nt of non-coding small RNAs were collected from two ES cell lines, six 4N-iPS cell lines and two 2N-iPS cell lines and sequenced in the invention. The cluster analysis method proves that the pluripotent stem cells with the tetraploid complementation ability have great difference from the 2N-iPs cells, for both the ES cells and the iPS cells; however, the 4N-ES cells and the 4N-iPS cells have no obvious difference (see Fig. 1). A further research found that 75 microRNAs had more than two-time expression changes between 2N and 4N cells, wherein 62 microRNAs were generally lowly expressed or not expressed in the 2N-iPS cell line, but were highly expressed in 4N-ES/4N-iPS cell lines generally.

### High activity of the imprinted region Dlk1-Dio3 is an important characterization of pluripotency

Fig. 2 shows all genes, microRNAs and other non-coding RNAs in the imprinted region Dlk1-Dio3 of mouse chromosome 12 (containing Dlk1 and Dio3 genes and the intergenic regions from this region to the upstream gene Begain and to the downstream gene Ppp2r5c). The expression of genes of this region is different in 2n-iPS cells and 4n-ES/iPS cells. Green rectangles are used to mark the genes and other non-coding RNAs with up-regulated expression in 4n-ES/iPS cells; genes with no expression differences are marked by grey rectangles outside the imprinted region; blank squares are used to mark undetected genes. Hairpin-shaped structure represents large amount of microRNAs generated by this region; the deeper the color is, the higher the expression level of the microRNA is in 4n-ES/iPS cells. Pentagons indicate other non-coding small RNAs; the shade of color and the change tendency of expression level thereof are consistent with the above microRNAs. All genes, microRNAs and other non-coding RNAs of the imprinted Dlk1-Dio3 region are in an activation state in 4n-ES/iPS cells.

It should be noted that most of the detected differentially expressed miRNAs are the lowly expressed microRNAs in 2N-iPS cells are located in the imprinted Dlk1-Dio3 region of mouse chromosome 12. This region is of about 1380kb, including five genes of Dlk1, Rtl1, 1110006E14Rik, B830012L14Rik and Dio3, three non-coding genes of Meg3, Rian and Mirg, a gene cluster of C/D box snoRNAs, 72 microRNAs and a plurality of other non-coding small RNAs (see Fig. 2). A previous research shows that the expression of the genes and microRNAs of this region is only in the mouse embryo and the adult-mouse brain, but their specific functions are not clear.

The foregoing five genes and three non-coding genes are as shown in Table 2.

**Table 2: Genes of the imprinted Dlk1-Dio3 region located on the long arm of mouse chromosome 12 and their genomic loci.**

| Gene Name | Genome locus (loci on chr12) |
|---|---|
| Dlk1 | 110691059-110698901 |
| Meg3 | 110779211-110809936 |
| Rtl1 | 110828379-110833613 |
| 1110006E14Rik | 110833609-110835408 |
| Rian | 110884339-110890480 |
| B830012L14Rik | 110933796-110936926 |
| Mirg | 110968996-110987668 |
| Dio3 | 111517470-111518918 |

The foregoing 72 microRNAs are as shown in Table 3:

**Table 3: MicroRNAs of the imprinted Dlk1-Dio3 region located on the long arm of mouse chromosome 12 and sequences thereof**

| | |
|---|---|
| mmu-miR-1188 | SEQ ID :UGGUGUGAGGUUGGGCCAGGA |
| mmu-miR-1193 | SEQ ID2:UAGGUCACCCGUUUUACUAUC |
| mmu-miR-1197 | SEQ ID3:UAGGACACAUGGUCUACUUCU |
| mmu-miR-127 | SEQ ID4:UCGGAUCCGUCUGAGCUUGGCU |
| mmu-miR-127* | SEQ IDS:CUGAAGCUCAGAGGGCUCUGAU |
| mmu-miR-134 | SEQ ID6:UGUGACUGGUUGACCAGAGGGG |
| mmu-miR-136 | SEQ ID7: ACUCCAUUUGUUUUGAUGAUGG |
| mmu-miR-136* | SEQ ID8:AUCAUCGUCUCAAAUGAGUCUU |
| mmu-miR-154 | SEQ ID9:UAGGUUAUCCGUGUUGCCUUCG |
| mmu-miR-154* | SEQ ID10:AAUCAUACACGGUUGACCUAUU |
| mmu-miR-299 | SEQ ID 11:UAUGUGGGACGGUAAACCGCUU |
| mmu-miR-299* | SEQ IDT2:UGGUUUACCGUCCCACAUACAU |
| mmu-miR-300 | SEQ ID13:UAUGCAAGGGCAAGCUCUCUUC |
| mmu-miR-300* | SEQ ID14:UUGAAGAGAGGUUAUCCUUUGU |
| mmu-miR-323-3p | SEQ ID15: CACAUUACACGGUCGACCUCU |
| mmu-miR-323-Sp | SEQ ID16: AGGUGGUCCGUGGCGCGUUCGC |
| mmu-miR-329 | SEQ ID17:AACACACCCAGCUAACCUUUUU |
| mmu-miR-337-3p | SEQ ID18: UUCAGCUCCUAUAUGAUGCCU |
| mmu-miR-337-5p | SEQ ID19: GAACGGCGUCAUGCAGGAGUU |
| mmu-miR-341 | SEQ ID20:UCGGUCGAUCGGUCGGUCGGU |
| mmu-miR-369-3p | SEQ ID21: AAUAAUACAUGGUUGAUCUUU |
| mmu-miR-3 69-5p | SEQ ID22: AGAUCGACCGUGUUAUAUUCGC |
| mmu-miR-370 | SEQ ID23:GCCUGCUGGGGUGGAACCUGGU |
| mmu-miR-376a | SEQ ID24:AUCGUAGAGGAAAAUCCACGU |
| mmu-miR-376a* | SEQ ID25:GGUAGAUUCUCCUUCUAUGAGU |
| mmu-miR-376b | SEQ ID26:AUCAUAGAGGAACAUCCACUU |
| mmu-miR-376b* | SEQ ID27:GUGGAUAUUCCUUCUAUGGUUA |
| mmu-miR-376c | SEQ ID28:AACAUAGAGGAAAUUUCACGU |
| mmu-miR-376c* | SEQ ID29:GUGGAUAUUCCUUCUAUGUUUA |
| mmu-miR-377 | SEQ ID30:AUCACACAAAGGCAACUUUUGU |
| mmu-miR-379 | SEQ ID31:UGGUAGACUAUGGAACGUAGG |
| mmu-miR-380-3p | SEQ ID32: UAUGUAGUAUGGUCCACAUCUU |
| mmu-miR-3 80-5p | SEQ ID33: AUGGUUGACCAUAGAACAUGCG |
| mmu-miR-381 | SEQ ID34:UAUACAAGGGCAAGCUCUCUGU |
| mmu-miR-3 82 | SEQ ID35:GAAGUUGUUCGUGGUGGAUUCG |
| mmu-miR-382* | SEQ ID36:UCAUUCACGGACAACACLTUUUU |
| mmu-miR-409-3p | SEQ ID37: GAAUGUUGCUCGGUGAACCCCU |
| mmu-miR-409-5p | SEQ ID38:AGGUUACCCGAGCAACUUUGCAU |
| mmu-miR-410 | SEQ ID39:AAUAUAACACAGAUGGCCUGU |
| mmu-miR-411 | SEQ ID40:UAGUAGACCGUAUAGCGUACG |
| mmu-miR-411* | SEQ ID41:UAUGUAACACGGUCCACUAACC |
| mmu-miR-412 | SEQ ID42:UUCACCUGGUCCACUAGCCG |
| mmu-miR-431 | SEQ ID43: UGUCUUGCAGGCCGUCAUGCA |
| mmu-miR-4331* | SEQ ID44:CAGGUCGUCUUGCAGGGCUUCU |
| mmu-miR-433 | SEQ ID4S:AUCAUGAUGGGCUCCUCGGUGU |
| mmu-miR-433* | SEQ ID46: UACGGUGAGCCUGUCAUUAUUC |
| mmu-miR-434-3p | SEQ ID47:UUUGAACCAUCACUCGACUCCU |
| mmu-miR-434-5p | SEQ ID48: GCUCGACUCAUGGUUUGAACCA |
| mmu-miR-485 | SEQ ID49:AGAGGCUGGCCGUGAUGAAUUC |
| mmu-miR-485* | SEQ ID50:AGUCAUACACGGCUCUCCUCUC |
| mrnu-miR-487b | SEQ ID51:AAUCGUACAGGGUCAUCCACUU |
| mmu-miR-493 | SEQ ID52: UGAAGGUCCUACUGUGUGCCAGG |
| mmu-miR-494 | SEQ ID53:UGAAACAUACACGGGAAACCUC |
| mmu-miR-495 | SEQ TD54:AAACAAACAUGGUGCACUUCUU |
| mmu-miR-496 | SEQ ID55:UGAGUAUUACAUGGCCAAUCUC |
| mmu-miR-539 | SEQ ID56:GGAGAAAUUAUCCUUGGUGUGU |
| mmu-miR-540-3p | SEQ ID57:AGGUCAGAGGUCGAUCCUGG |
| mmu-miR-540-5p | SEQ ID58: CAAGGGUCACCCUCUGACUCUGU |
| mmu-miR-541 | SEQ ID59:AAGGGAUUCUGAUGUUGGUCACACU |
| mrnu-miR-543 | SEQ ID60:AAACAUUCGCGGUGCACUUCUU |
| mmu-miR-544 | SEQ ID61: AUUCUGCAUUUUUAGCAAGCUC |
| mmu-miR-665 | SEQ ID62:ACCAGGAGGCUGAGGUCCCU |
| mmu-miR-666-3p | SEQ ID63: GGCUGCAGCGUGAUCGCCUGCU |
| mmu-miR-666-5p | SEQ ID64:AGCGGGCACAGCUGUGAGAGCC |
| mmu-miR-667 | SEQ ID6S:UGACACCUGCCACCCAGCCCAAG |
| mmu-miR-668 | SEQ ID66: UGUCACUCGGCUCGGCCCACUACC |
| mmu-miR-673-3p | SEQ ID67:UCCGGGGCUGAGUUCUGUGCACC |
| mmu-miR-673-5p | SEQ ID68:CUCACAGCUCUGGUCCUUGGAG |
| mmu-miR-679 | SEQ ID69:GGACUGUGAGGUGACUCUUGGU |
| mmu-miR-758 | SEQ ID70:UUUGUGACCUGGUCCACUA |
| mmu-miR-770-3p | SEQ ID71:CGUGGGCCUGACGUGGAGCUGG |
| mmu-miR-770-5p | SEQ ID72:AGCACCACGUGUCUGGGCCACG |

The microRNAs of the Dlk1-Dio3 region mentioned above generally are lowly expressed in the 2N-iPS cell lines; compared to 2N-iPS cells, the microRNAs are generally highly expressed in the 4N-ES/iPS cell lines. Another two microRNAs mir-342 and mir-345 of adjacent regions also have similar tendency (see Fig.3A). More notably, massively endogenous siRNAs of this region also have the same expression trend (see Fig. 3B). In addition, using expression profiling microarray to analyze transcription abundance, it is discovered that all genes of this region except for B830012L14Rik are highly expressed in the 4N-ES/iPS cell lines but are suppressed in the 2N-iPS cells (see Fig. 3C). Therefore, the inventors conclude that high activity of the imprinted Dlk1-Dio3 region is an important characterization of true pluripotency.

### Small expression differences of partially reprogrammed cell lines with or without germ-line chimeric ability

In this research, the pluripotency of two 2n iPS cell lines has difference too; IP20D-3 has a germ-line chimeric ability, while IP36D-3 does not have this ability. As deduced by the inventors, although the encoded microRNAs of the Dlk1-Dio3 region are lowly or not expressed in the two 2n-iPS cell lines, the expression level in the IP20D-3 generally is higher than that in the IP36D-3 among these limited expressions (see Fig. 4). Corresponding to the germ-line chimeric ability of the IP20D-3, it is very likely that the higher expression of microRNAs from this imprinted region endowed this cell line with a higher developmental potentiality.

### Activated microRNA clusters positive-feedback regulate the PRC2 complex and caused demethylation of the Dlk1-Dio3 region

The expression profiling microarray of 2n iPS cells and ES cells and 4n iPS cell lines shows that 1638 genes have expression up-regulated in ES and 4n iPS cell lines relative to 2n iPS cell lines and 3467 genes have expression down-regulated. Since the microRNA generally has a negative regulation on the target gene thereof, the genes with expression down-regulated in ES and 4n iPS cells should include the target genes of the microRNAs cluster identified and obtained by the inventors. The inventors analyzed the 3'UTR regions of mRNAs with at least two loci which can be in complement combination with the 2-8 nucleic acid seed regions of microRNA 5' end, and found that 28 microRNAs, which are moderately or highly expressed, have 717 potential target genes with down-regulated expression in the ES and 4n iPS cell lines.

Gene Ontology (GO) analysis shows that the target genes deducted from the microRNAs with high abundance are related to growth, differentiation and metabolism, and development process regulation and other phenotypes, and that the microRNA cluster highly expressed in fully pluripotent cells may function by suppressing development related genes.

Signalling pathway analysis shows that three elements (HDAC2, RBAP48 and EED) forming the Polycomb Repressive Complex 2 (PRC2) are predicted microRNA target genes. The PRC2 can trigger trimethylation of Lysine at position 27 of histone H3, thereby causing gene silencing. Before the PRC2 triggers methylation, it is needed to activate Histone Deacetylase 2 (HDAC2) to remove the acetylation modification of histone H3. Our research result indicates that, in ES and 4n iPS cells, the expression of encoded microRNAs of the Dlk1-Dio3 region suppresses the expression of HDAC2, RBAP48 and EED, thereby resulting in the reduction of formation of PRC2 and demethylation of genome. In ES and iPS cells, the reduction of formation of PRC2 would cause demethylation of the Dlk1-Dio3 region, which coincides with our research result that the expression of genes and microRNAs is increased. (See Fig. 5)The expression of microRNAs further suppresses the formation of PRC2 by targeting the transcription of hdac2, rbap48 and eed, and finally a positive feedback regulation signalling pathway is formed.

Fig. 5 shows a mechanism sketch model of microRNAs of the Dlk1-Dio3 region regulating a PRC2 complex. A. The PRC2 complex suppresses the expression of genes and microRNAs through tri-methylation of Histone H3K27 locus. B. In partially pluripotent stem cells, the expression of microRNAs of the Dlk1-Dio3 region is suppressed by the methylation of Histone H3K27 mediated by the PRC2 complex. In fully pluripotent stem cells, microRNAs of the Dlk1-Dio3 region are in an activation state. Therefore, the mRNA used for guiding the synthesis of the three proteins of HDAC2, RBAP48 and EED can not function normally; cells can not form the PRC2 complex, thereby causing the demethylation of the Dlk1-Dio3 region and further facilitating the expression of microRNAs of the Dlk1-Dio3 region to form a positive feedback regulation pathway.

### Results of function verification experiment:

The expression of genes and microRNAs of the Dlk1-Dio3 region is detected in six 4N-ES cell lines, seven 4N-iPS cell lines, four GLT-iPS cell lines, one 2N-iPS cell and one 2N-ES cell by methods of real-time qPCR and Northern blot hybridization. The result shows that the genes and microRNAs of the Dlk1-Dio3 region are highly expressed in the six 4N-ES cell lines and seven 4N-iPS cell lines; are moderately expressed in the four GLT-iPS cell lines, without a high expression level; and are not or lowly expressed in the 2N-iPS cells and 2N-ES cells. It is proved that the genes and microRNAs of the Dlk1-Dio3 region regulate the pluripotency of cells. If the genes and microRNAs of this region are highly expressed in a cell, it is indicated that this cell has a better developmental potentiality.

### References

Hu, B.Y, Weick, J.P., Yu, J., Ma, L.X., Zhang, X.Q., Thomson, J.A., and Zhang, S.C. Neural differentiation of human induced pluripotent stem cells follows developmental principles but with variable potency. Proc Natl Acad Sci U S A 107, 4335-4340.
Judson, R.L., Babiarz, J.E., Venere, M., and Blelloch, R. (2009). Embryonic stem cell-specific microRNAs promote induced pluripotency. Nat Biotechnol 27, 459-461.
Lin, S.P., Youngson, N., Takada, S., Seitz, H., Reik, W., Paulsen, M., Cavaille, J., and Ferguson-Smith, A.C. 2003. Asymmetric regulation of imprinting on the maternal and paternal chromosomes at the Dlk1-Gtl2 imprinted cluster on mouse chromosome 12. Nat. Genet. 35: 97-102.
Melton, C., Judson, R.L., and Blelloch, R. Opposing microRNA families regulate self-renewal in mouse embryonic stem cells. Nature 463, 621-626.
Seitz H, Royo H, Bortolin ML et al. A large imprinted microRNA gene cluster at the mouse Dlk1-Gtl2 domain. Genome Res 2004;14: 1741-1748.
Seitz, H., Youngson, N., Lin, S.P., Dalbert, S., Paulsen, M., Bachellerie, J.P., Ferguson-Smith, A.C., and Cavaille, J. 2003a. Imprinted microRNA genes transcribed antisense to a reciprocally imprinted retrotransposon-like gene. Nat. Genet. 34: 261-262.
Takahashi, K., and Yamanaka, S. (2006). Induction of pluripotent stem cells from mouse embryonic and adult fibroblast cultures by defined factors. Cell 126, 663-676.
Takahashi, K., Tanabe, K., Ohnuki, M., Narita, M., Ichisaka, T., Tomoda, K., and Yamanaka, S. (2007). Induction of pluripotent stem cells from adult human fibroblasts by defined factors. Cell 131, 861-872.
Yu, J.Y, Vodyanik, M.A., Smuga-Otto, K., Antosiewicz-Bourget, J., Frane, J.L., Tian, S., Nie, J., Jonsdottir, G.A., Ruotti, V, Stewart, R., et al. (2007). Induced pluripotent stem cell lines derived from human somatic cells. Science 318, 1917-1920.
Yvonne M.-S. Tay, Wai-Leong Tam, Yen-sin Ang, Philip M.Gaughwin, Henry Yang, Whijia Wang, Rubing Liu, Joshy George, Huck-hui Ng, Ranjan J. Perera, Thomas Lufkin, Isidore Rigoutsos, Andrew M. Thomson, Bing Lim. 2008. MicroRNA-134 modulates the differentiation of mouse embryonic stem cells, where it causes post-transcriptional attenuation of Nanog and LRH1. Stem Cells 2008;26:17-29
Zhao, X.Y, Li, W., Lv, Z., Liu, L., Tong, M., Hai, T., Hao, J., Guo, C.L., Ma, Q.W., Wang, L., et al. (2009). iPS cells produce viable mice through tetraploid complementation. Nature 461, 86-U88.

## Claims

1. Key genes, microRNAs, other non-coding RNAs and a combination thereof for identifying and regulating pluripotency of cells, **characterized in that** they are highly expressed in full pluripotent stem cells but are significantly suppressed or silenced in partially pluripotent stem cells.

2. The genes, microRNAs, other non-coding RNAs and combination thereof according to claim 1, **characterized in that** the expression level is obviously higher in the pluripotent stem cells with the ability to produce germ-line chimeric animals than in the pluripotent stem cells without the ability to produce germ-line chimeric animals; and the expression level is positively correlated to the pluripotent level of cells.

3. The genes, microRNAs, other non-coding RNAs and combination thereof according to claim 1 or 2, **characterized in that** the genes, microRNAs and other non-coding RNAs include the genes, microRNAs and other non-coding RNAs of a chromosomal imprinted region Dlk1-Dio3 located on the long arm of mouse chromosome 12, and homologous genes, microRNAs and other non-coding RNAs having 70-100% homology with them in polynucleotide sequences in genomic syntenic regions of other mammals; the chromosome imprinted region Dlk1-Dio3 contains Dlk1 and Dio3 genes and intergenic regions from this region to the upstream gene Begain and to the downstream gene Ppp2r5c.

4. The genes, microRNAs, other non-coding RNAs and combination thereof according to claim 3, **characterized in that** the chromosomal imprinted region Dlk1-Dio3 is of about 1380kb.

5. The genes, microRNAs, other non-coding RNAs and combination thereof according to claim 1 or 2, **characterized in that** the microRNA includes all microRNAs listed in SEQ ID 1-72.

6. The genes, microRNAs, other non-coding RNAs and combination thereof according to claim 3, **characterized in that** the genes, microRNAs and other non-coding RNAs include five genes of Dlk1, Rtl1, 1110006E14Rik, B830012L14Rik and Dio3, three non-coding genes of Meg3, Rian and Mirg, a gene cluster of C/D box snoRNAs, and all encoded microRNAs and other non-coding RNAs of this region.

7. Uses of the genes, microRNAs, other non-coding RNAs and combination thereof according to any one of claims 1 to 6 in identifying the pluripotent status of stem cells, in regulating pluripotency of cells, in typing stem cells, regulating pluripotency of cells and pluripotent states and levels of cells, treating diseases, or developing drug targets for tumor treatment or antitumor drugs.

## Amended claims

### Amended claims under Art. 19.1 PCT

**1.** Uses of a microRNA cluster in a chromosomal imprinted Dlk1-Dio3 region located on the long arm of mouse chromosome 12 or any microRNA therein or a microRNA combination thereof and homologous genes of any microRNA above in other species or a combination thereof in identifying or regulating pluripotent levels of Embryonic Stem cells (ES cells) and induced Pluripotent Stem cells (iPS cells), **characterized in that** the microRNA cluster is highly expressed in full pluripotent ES cells and iPS cells but is significantly suppressed or silenced in partially pluripotent ES cells and iPS cells.

**2.** The uses according to claim 1, **characterized in that** the expression level of the microRNA cluster is positively correlated to the pluripotent level of the ES cells and iPS cells, that is, the expression level in the ES cells and iPS cells which can form germ-line chimera is obviously higher than that in the ES cells and iPS cells which can not form germ-line chimera.

**3.** The uses according to claim 1 or 2, **characterized in that** the microRNA includes all microRNAs listed in SEQ ID 1-72.

**4.** Uses of the microRNA cluster or any microRNA therein or microRNA combination thereof and homologous genes of any microRNA above in other species or combination thereof according to any one of claims 1 to 3 in isolating, proliferating and typing ES cells and iPS cells and their uses in the treatment of diseases relating to the ES cells and iPS cells.

**5.** Uses of Rtl1, Meg3, Rian, 1110006E14Rik, B830012L14Rik and Mirg genes in a chromosome imprinted region Dlk1-Dio3 located on the long arm of mouse chromosome 12 or a combination thereof and homologous genes of the genes above in other species or a combination thereof in identifying or regulating pluripotent levels of ES cells and iPS cells, **characterized in that** the genes are highly expressed in full pluripotent ES cells and iPS cells but are significantly suppressed or silenced in partially pluripotent ES cells and iPS cells.

**6.** The uses according to claim 5, **characterized in that** the expression level of the genes is positively correlated to the pluripotent level of the ES cells and iPS cells, that is, the expression level in the ES cells and iPS cells which can form germ-line chimera is obviously higher than that in the ES cells and iPS cells which can not form germ-line chimera.

**7.** Uses of the genes or combination thereof and homologous genes of said genes in other species or combination thereof according to any one of claims 5 to 6 in isolating, proliferating and typing ES cells and iPS cells and their uses in the treatment of diseases relating to the ES cells and iPS cells.


SEQUENCE LISTING

<110>  INSTITUTE OF ZOOLOGY, CHINESE ACADEMY OF SCIENCES
       INSTITUTE OF GENETICS AND DEVELOPMENTAL BIOLOGY, CHINESE ACADEMY OF
SCIENCES

<120>  Key genes, microRNAs, other non-coding RNAs and combination thereof
for identifying and regulating the pluripotent status of cells

<130>  P63476DWYJY

<140>  201010142746 8
<141>  2010-04-07

<160>  72

<170>  PatentIn version 3.2

<210>  1
<211>  21
<212>  RNA
<213>  Mus musculus

<400>  1
uggugugagg uugggccagg a                                                21


<210>  2
<211>  21
<212>  RNA
<213>  Mus musculus

<400>  2
uaggucaccc guuuuacuau c                                                21


<210>  3
<211>  21
<212>  RNA
<213>  Mus musculus

<400>  3
uaggacacau ggucuacuuc u                                                21


<210>  4
<211>  22
<212>  RNA
<213>  Mus musculus

<400>  4
ucggauccgu cugagcuugg cu                                               22


<210>  5
<211>  22
<212>  RNA
<213>  Mus musculus

<400>  5
cugaagcuca gagggcucug au                                               22


<210>  6
<211>  22

<212>  RNA
<213>  Mus musculus

<400>  6
ugugacuggu ugaccagagg gg                                              22


<210>  7
<211>  22
<212>  RNA
<213>  Mus musculus

<400>  7
acuccauuug uuuugaugau gg                                              22


<210>  8
<211>  22
<212>  RNA
<213>  Mus musculus

<400>  8
aucaucgucu caaaugaguc uu                                              22


<210>  9
<211>  22
<212>  RNA
<213>  Mus musculus

<400>  9
uagguuaucc guguugccuu cg                                              22


<210>  10
<211>  22
<212>  RNA
<213>  Mus musculus

<400>  10
aaucauacac gguugaccua uu                                              22


<210>  11
<211>  22
<212>  RNA
<213>  Mus musculus

<400>  11
uaugugggac gguaaaccgc uu                                              22


<210>  12
<211>  22
<212>  RNA
<213>  Mus musculus

<400>  12
ugguuuaccg ucccacauac au                                              22


<210>  13
<211>  22
<212>  RNA
<213>  Mus musculus

<400>  13
uaugcaaggg caagcucucu uc                                              22


<210>  14
<211>  22
<212>  RNA
<213>  Mus musculus

<400>  14
uugaagagag guuauccuuu gu                                              22


<210>  15
<211>  21
<212>  RNA
<213>  Mus musculus

<400>  15
cacauuacac ggucgaccuc u                                               21


<210>  16
<211>  22
<212>  RNA
<213>  Mus musculus

<400>  16
   aggugguccg uggcgcguuc gc                                             22


<210>  17
<211>  22
<212>  RNA
<213>  Mus musculus

<400>  17
aacacaccca gcuaaccuuu uu                                              22


<210>  18
<211>  21
<212>  RNA
<213>  Mus musculus

<400>  18
uucagcuccu auaugaugcc u                                               21


<210>  19
<211>  21
<212>  RNA
<213>  Mus musculus

<400>  19
gaacggcguc augcaggagu u                                               21


<210>  20
<211>  21
<212>  RNA
<213>  Mus musculus

<400>  20

ucggucgauc ggucggucgg u                                                21

<210>  21
<211>  21
<212>  RNA
<213>  Mus musculus

<400>  21
aauaauacau gguugaucuu u                                                21

<210>  22
<211>  22
<212>  RNA
<213>  Mus musculus

<400>  22
agaucgaccg uguuauauuc gc                                               22

<210>  23
<211>  22
<212>  RNA
<213>  Mus musculus

<400>  23
gccugcuggg guggaaccug gu                                               22

<210>  24
<211>  21
<212>  RNA
<213>  Mus musculus

<400>  24
aucguagagg aaaauccacg u                                                21

<210>  25
<211>  22
<212>  RNA
<213>  Mus musculus

<400>  25
gguagauucu ccuucuauga gu                                               22

<210>  26
<211>  21
<212>  RNA
<213>  Mus musculus

<400>  26
aucauagagg aacauccacu u                                                21

<210>  27
<211>  22
<212>  RNA
<213>  Mus musculus

<400>  27
guggauauuc cuucuauggu ua                                               22

<210>  28
<211>  21
<212>  RNA
<213>  Mus musculus

<400>  28
aacauagagg aaauuucacg u                                                21


<210>  29
<211>  22
<212>  RNA
<213>  Mus musculus

<400>  29
guggauauuc cuucuauguu ua                                               22


<210>  30
<211>  22
<212>  RNA
<213>  Mus musculus

<400>  30
aucacacaaa ggcaacuuuu gu                                               22


<210>  31
<211>  21
<212>  RNA
<213>  Mus musculus

<400>  31
ugguagacua uggaacguag g                                                21


<210>  32
<211>  22
<212>  RNA
<213>  Mus musculus

<400>  32
uauguaguau gguccacauc uu                                               22


<210>  33
<211>  22
<212>  RNA
<213>  Mus musculus

<400>  33
augguugacc auagaacaug cg                                               22


<210>  34
<211>  22
<212>  RNA
<213>  Mus musculus

<400>  34
uauacaaggg caagcucucu gu                                               22


<210>  35

<211>  22
<212>  RNA
<213>  Mus musculus

<400>  35
gaaguuguuc gugguggauu cg                                                22


<210>  36
<211>  22
<212>  RNA
<213>  Mus musculus

<400>  36
ucauucacgg acaacacuuu uu                                                22


<210>  37
<211>  22
<212>  RNA
<213>  Mus musculus

<400>  37
gaauguugcu cggugaaccc cu                                                22


<210>  38
<211>  23
<212>  RNA
<213>  Mus musculus

<400>  38
agguuacccg agcaacuuug cau                                               23


<210>  39
<211>  21
<212>  RNA
<213>  Mus musculus

<400>  39
aauauaacac agauggccug u                                                 21


<210>  40
<211>  21
<212>  RNA
<213>  Mus musculus

<400>  40
uaguagaccg uauagcguac g                                                 21


<210>  41
<211>  22
<212>  RNA
<213>  Mus musculus

<400>  41
uauguaacac gguccacuaa cc                                                22


<210>  42
<211>  20
<212>  RNA

<213>  Mus musculus

<400>  42
uucaccuggu ccacuagccg                                                    20


<210>  43
<211>  21
<212>  RNA
<213>  Mus musculus

<400>  43
ugucuugcag gccgucaugc a                                                  21


<210>  44
<211>  22
<212>  RNA
<213>  Mus musculus

<400>  44
caggucgucu ugcagggcuu cu                                                 22


<210>  45
<211>  22
<212>  RNA
<213>  Mus musculus

<400>  45
aucaugaugg gcuccucggu gu                                                 22


<210>  46
<211>  22
<212>  RNA
<213>  Mus musculus

<400>  46
uacggugagc cugucauuau uc                                                 22


<210>  47
<211>  22
<212>  RNA
<213>  Mus musculus

<400>  47
uuugaaccau cacucgacuc cu                                                 22


<210>  48
<211>  22
<212>  RNA
<213>  Mus musculus

<400>  48
gcucgacuca ugguuugaac ca                                                 22


<210>  49
<211>  22
<212>  RNA
<213>  Mus musculus

<400>  49
agaggcuggc cgugaugaau uc                                                22

<210>  50
<211>  21
<212>  RNA
<213>  Mus musculus

<400>  50
agucauacac ggcucuccuc u                                                 21

<210>  51
<211>  22
<212>  RNA
<213>  Mus musculus

<400>  51
aaucguacag ggucauccac uu                                                22

<210>  52
<211>  23
<212>  RNA
<213>  Mus musculus

<400>  52
ugaagguccu acugugugcc agg                                               23

<210>  53
<211>  22
<212>  RNA
<213>  Mus musculus

<400>  53
ugaaacauac acgggaaacc uc                                                22

<210>  54
<211>  22
<212>  RNA
<213>  Mus musculus

<400>  54
aaacaaacau ggugcacuuc uu                                                22

<210>  55
<211>  22
<212>  RNA
<213>  Mus musculus

<400>  55
ugaguauuac auggccaauc uc                                                22

<210>  56
<211>  22
<212>  RNA
<213>  Mus musculus

<400>  56
ggagaaauua uccuuggugu gu                                                22

<210>  57
<211>  20
<212>  RNA
<213>  Mus musculus

<400>  57
aggucagagg ucgauccugg                                                    20


<210>  58
<211>  23
<212>  RNA
<213>  Mus musculus

<400>  58
caagggucac ccucugacuc ugu                                                23


<210>  59
<211>  25
<212>  RNA
<213>  Mus musculus

<400>  59
aagggauucu gauguugguc acacu                                              25


<210>  60
<211>  22
<212>  RNA
<213>  Mus musculus

<400>  60
aaacauucgc ggugcacuuc uu                                                 22


<210>  61
<211>  22
<212>  RNA
<213>  Mus musculus

<400>  61
auucugcauu uuuagcaagc uc                                                 22


<210>  62
<211>  20
<212>  RNA
<213>  Mus musculus

<400>  62
accaggaggc ugaggucccu                                                    20


<210>  63
<211>  22
<212>  RNA
<213>  Mus musculus

<400>  63
ggcugcagcg ugaucgccug cu                                                 22

<210>  64
<211>  22
<212>  RNA
<213>  Mus musculus

<400>  64
agcgggcaca gcugugagag cc                                                22


<210>  65
<211>  23
<212>  RNA
<213>  Mus musculus

<400>  65
ugacaccugc cacccagccc aag                                               23


<210>  66
<211>  24
<212>  RNA
<213>  Mus musculus

<400>  66
ugucacucgg cucggcccac uacc                                              24


<210>  67
<211>  23
<212>  RNA
<213>  Mus musculus

<400>  67
uccggggcug aguucugugc acc                                               23


<210>  68
<211>  22
<212>  RNA
<213>  Mus musculus

<400>  68
cucacagcuc ugguccuugg ag                                                22


<210>  69
<211>  22
<212>  RNA
<213>  Mus musculus

<400>  69
ggacugugag gugacucuug gu                                                22


<210>  70
<211>  19
<212>  RNA
<213>  Mus musculus

<400>  70
uuugugaccu gguccacua                                                    19


<210>  71
<211>  22

<212>  RNA
<213>  Mus musculus

<400>  71
cgugggccug acguggagcu gg                                                22


<210>  72
<211>  22
<212>  RNA
<213>  Mus musculus

<400>  72
agcaccacgu gucugggcca cg                                                22